(19) Europäisches Patentamt
European Patent Office
Office européen des brevets

(11) EP 2 188 233 B1

(12) # EUROPÄISCHE PATENTSCHRIFT

(45) Veröffentlichungstag und Bekanntmachung des Hinweises auf die Patenterteilung:
**13.07.2016 Patentblatt 2016/28**

(21) Anmeldenummer: **08787271.9**

(22) Anmeldetag: **15.08.2008**

(51) Int Cl.:
***C07B 63/00*** (2006.01) ***C07D 233/54*** (2006.01)
***C07C 51/44*** (2006.01) ***C07C 53/122*** (2006.01)
***C07C 53/10*** (2006.01)

(86) Internationale Anmeldenummer:
**PCT/EP2008/060743**

(87) Internationale Veröffentlichungsnummer:
**WO 2009/027250 (05.03.2009 Gazette 2009/10)**

(54) **DESTILLATION IONISCHER FLÜSSIGKEITEN**

DISTILLATION OF IONIC LIQUIDS

DISTILLATION DE FLUIDES IONIQUES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **31.08.2007 DE 102007041416**

(43) Veröffentlichungstag der Anmeldung:
**26.05.2010 Patentblatt 2010/21**

(73) Patentinhaber: **BASF SE**
**67056 Ludwigshafen am Rhein (DE)**

(72) Erfinder:
- **MASSONNE, Klemens**
**67098 Bad Dürkheim (DE)**
- **SIEMER, Michael**
**68159 Mannheim (DE)**
- **MORMANN, Werner**
**57076 Siegen (DE)**
- **LENG, Wei**
**57076 Siegen (DE)**

(74) Vertreter: **BASF IP Association**
**BASF SE**
**ZRX-C6**
**67056 Ludwigshafen (DE)**

(56) Entgegenhaltungen:
**WO-A-91/14678 WO-A-2005/068404**

- **MACFARLANE D R ET AL: "Lewis base ionic liquids" CHEMICAL COMMUNICATIONS - CHEMCOM, ROYAL SOCIETY OF CHEMISTRY, GB, 3. März 2006 (2006-03-03), Seiten 1905-1917, XP002483141 ISSN: 1359-7345 in der Anmeldung erwähnt**
- **X. HE ETAL.: "Ionic-tag-assisted oligosaccharide synthesis" SYNTHESIS, Nr. 10, 2006, Seiten 1645-1651, XP002521376**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 188 233 B1

Printed by Jouve, 75001 PARIS (FR)

**Beschreibung**

Die Erfindung betrifft ein Verfahren zur Destillation von Gemischen, welche Salze mit einem Schmelzpunkt kleiner 200°C bei 1 bar (ionische Flüssigkeiten) enthalten, welches dadurch gekennzeichnet ist, dass

- das Kation der ionischen Flüssigkeit ein heterocyclisches Ringsystem mit mindestens einem Stickstoffatom enthält und alle Stickstoffatome des heterocyclischen Ringsystems eine organische Gruppe als Substituenten haben
- es sich bei dem Anion der ionischen Flüssigkeit um eine Verbindung mit mindestens einer Carboxylatgruppe (kurz Carboxylat) oder mindestens einer Phosphatgruppe (kurz Phosphat) handelt und
- der Gehalt an ionischer Flüssigkeit im Gemisch mindestens 5 Gew.% beträgt,
- der Gehalt an leicht flüchtige Verbindungen (Siedepunkt kleiner als 120°C, 1 bar) 0 bis 10 Gew.%, bezogen auf das Gemisch, beträgt
- bei der Destillation der Abstand der Fläche, über welche die Destillationswärme zugeführt wird (Verdampferoberfläche) zu der Fläche, an der die Kondensation erfolgt (Kondensatoroberfläche), an mindestens einem Punkt geringer als 50 cm ist, wobei die Verdampfer- und Kondensatoroberfläche selbst mindestens eine Längenabmessung größer 50 cm haben,
- die ionische Flüssigkeit als Destillat von der Kondensatoroberfläche abgezogen wird und
- es sich um eine Molekulardestillation handelt.

Salze mit einem Schmelzpunkt kleiner 200°C, insbesondere mit einem Schmelzpunkt kleiner 100°C, werden als ionische Flüssigkeiten bezeichnet. Von besonderem Interesse sind ionische Flüssigkeiten, welche bereits bei Raumtemperatur flüssig sind. Derartige ionische Flüssigkeiten galten lange Zeit als nicht destillierbar, da angenommen wurde, dass sie letztlich keinen Dampfdruck besitzen.

Im Februar 2006 veröffentlichten Martyn J. Earle, Jose M.S.S. Esperanca et al in Nature, Vol 439, 2006, Seite 831 bis 834 einen Artikel über die Destillation flüchtiger ionischer Flüssigkeiten in einer Kugelrohr-apparatur. Ionische Flüssigkeiten mit Halogen-iden, Sulfaten oder Carboxylaten zersetzen sich jedoch und konnten nicht destilliert werden.

WO 2005/068404 beschreibt die Destillation von ionischen Flüssigkeiten, auch mit Halogeniden und Acetaten als Anion. Wesentlich ist dabei, dass die ionischen Flüssigkeiten aufgrund einer Gleichgewichtsreaktion auch als neutrale Verbindungen, das heißt nicht als Salz, vorliegen können. Durch die Destillation werden diese neutralen Verbindungen entfernt. Durch ständig neue Einstellung des Gleichgewichtszustandes destilliert die gesamte ionische Flüssigkeit in Form der neutralen Verbindungen. Bei ionischen Flüssigkeiten mit Stickstoff-haltigen, heterocyclischen Ringsystemen als Kation und z.B. Halogeniden bzw. Carboxylaten als Anion kann es nur zur Ausbildung eines entsprechenden Gleichgewichtszustandes kommen, wenn mindestens ein Stickstoffatom des Ringsystems nicht durch eine organische Gruppe substituiert ist und so für eine Gleichgewichtsreaktion mit dem Anion zur Verfügung steht. Entsprechend werden in den Beispielen der WO 2005/068404 nur die Chloride des 1-Ethylimidazol oder 1-Methylimidazol destilliert.

Auch aus Douglas R. MacFarlane, Jennifer M. Pringle et al., Chem.Commun., 2006, Seite 1905 bis 1917 ist eine Destillation von ionischen Flüssigkeiten bekannt. Hier beruht die Destillierbarkeit auf einer Gleichgewichtsreaktion bei der das Kation und Anion der ionischen Flüssigkeit als neutrale Säure und Base vorliegen. Wie vorstehend beschrieben, werden die neutralen Verbindungen dem Gleichgewichtszustand entzogen und destilliert. Auf diese Weise können Imidazolium-acetate destilliert werden, bei denen Stickstoffatom des heterocyclischen Ringsystems in protonierter Form vorliegt (HMIM-acetat in Tabelle 4 des Artikels).

Ionische Flüssigkeiten werden bei ihrer Verwendung im allgemeinen nicht verbraucht, sondern nur verunreinigt. Da es sich um hochpreisige Wertstoffe handelt, besteht ein Bedarf an besonders effektiven und günstigen Verfahren zur Aufarbeitung und Abtrennung der ionischen Flüssigkeiten aus den bei der Verwendung erhaltenen Gemischen. Bei der Verwendung von ionischen Flüssigkeiten zur Auflösung von Zellulose entstehen z.B. Gemische, welche Lignine oder Cellulosederivate enthalten. Darüber hinaus sind kostengünstige Herstellverfahren für ionische Flüssigkeiten bekannt, bei denen aber schwerflüchtige Nebenprodukte entstehen, die erhaltenen Reaktionsprodukte zeigen aufgrund dieser Nebenprodukte eine Verfärbung und erscheinen im allgemeinen schwarz. Derartige Herstellverfahren sind z.B. in WO 2005/021484 (Carbonatmethode) oder in WO 91/14678 (Arduengo - verfahren) beschrieben. Auch hier besteht ein Bedarf an besonders effektiven und günstigen Verfahren zur Aufarbeitung und Abtrennung der ionischen Flüssigkeiten aus den bei der Herstellung erhaltenen Gemischen.

Aufgabe der vorliegenden Erfindung ist daher ein einfaches und effektives Verfahren zur Reinigung oder Aufarbeitung von ionischen Flüssigkeiten bzw. der bei der Herstellung und/oder Verwendung erhaltenen Gemischen.

Demgemäß wurde das eingangs definierte Verfahren gefunden.

Zur ionischen Flüssigkeit

Bei der erfindungsgemäßen ionischen Flüssigkeit handelt es sich um ein Salz aus mindestens einem Kation

und mindestens einem Anion, welches bei Normaldruck (1 bar) einen Schmelzpunkt kleiner 200°C, insbesondere kleiner 100°C, vorzugsweise kleiner 75°C hat. Ganz besonders bevorzugt handelt es sich um ein bei Raumtemperatur (21°C) und Normaldruck (1 bar) flüssiges Salz.

Bei dem Kation der ionischen Flüssigkeit handelt es sich erfindungsgemäß um ein heterocyclisches Ringsystem mit mindestens einem Stickstoffatom als Bestandteil des Ringsystems. Alle Stickstoffatome des Ringsystems tragen eine organische Gruppe als Substituenten. Eine Protonierung dieser Stickstoffatome ist daher nicht möglich. Bei dem Substituenten handelt es sich vorzugsweise um eine organische Gruppe, welche 1 bis 20 C- Atome, insbesondere 1 bis 10 C-Atome enthält. Besonders bevorzugt handelt es sich um eine Kohlenwasserstoffgruppe, welche keine weiteren Heteroatome aufweist, z.B. um eine gesättigte oder ungesättigte aliphatische Gruppe, eine aromatische Gruppe oder eine Kohlenwasserstoffgruppe, welche sowohl aromatische als auch aliphatische Bestandteile aufweist. Ganz besonders bevorzugt handelt es sich um eine C1 bis C10 Alkylgruppe, C1 bis C10 Alkenylgrupe, z.B. eine Allylgruppe, eine Phenylgruppe oder eine Benzylgruppe.

In einer besonderen Ausführungsform handelt es sich um eine C1 bis C10, insbesondere eine C1 bis C4 Alkylgruppe, z.B. eine Methylgruppe, Ethylgruppe, Propylgruppe, i-Propylgruppe, n-Butylgruppe.

Vorzugsweise handelt es sich um ein aromatisches heterocyclisches Ringsystem.

Bei dem Kation handelt es sich vorzugsweise um ein Derivat des Imidazoliums, des Pyrazoliums oder des Pyridiniums.

Besonders bevorzugt handelt es sich bei dem Kation um ein Derivat des Imidazoliums.

Bei dem Anion der ionischen Flüssigkeit handelt es sich um eine Verbindung mit mindestens einer Carboxylatgruppe (kurz Carboxylat) oder mindestens einer Phosphatgruppe (kurz Phosphat).

Als Phosphate seien $PO_4^{3-}$ oder organische Verbindungen mit einer Phosphatgruppe, insbesondere Dialkylphosphate, genannt. Besonders bevorzugte Phosphate sind C1-C4 Dialkylphosphate, z.B. Dimethylphosphat und insbesondere Diethylphosphat.

Bevorzugte Anionen sind die Carboxylate.

Als Carboxylate seien insbesondere organische Verbindungen mit 1 bis 20C-Atomen, vorzugsweise 1 bis 10 C- Atomen genannt, die ein bis drei, vorzugsweise ein oder zwei, besonders bevorzugt eine Carboxylatgruppe enthalten.

Es kann sich dabei sowohl um aliphatische als auch um aromatische Verbindungen handeln, wobei unter den aromatischen Verbindungen solche verstanden werden, die aromatische Gruppen enthalten. Die aliphatischen oder aromatischen Verbindungen können gegebenenfalls weitere funktionelle Gruppen, z.B. Hydroxylgruppen, Carbonylgruppen oder Ethergrupen enthalten oder sonstige Heteroatome, insbesondere Halogene wie Fluor, Chlor oder Brom, vorzugsweise Fluor als Substituenten enthalten.

Ganz besonders bevorzugt sind aliphatische oder aromatische Verbindungen, die außer den Sauerstoffatomen der Carboxylatgruppe keine weiteren funktionellen Gruppen oder Heteroatome enthalten.

Als Verbindungen mit zwei Carboxylatgruppen seien z.B. die Anionen der Phthalsäure, der Isophthalsäure, der C2 bis C6 Dicarbonsäuren, z.B. Oxalsäure, Malonsäure, Bernsteinsäure, Glutarsäure oder Adipinsäure genannt.

Als Verbindungen mit einer Carboxylatgruppe seien die Anionen von aromatischen, aliphatischen, gesättigten oder ungesättigten C1 bis C20 Carbonsäuren, insbesondere Alkancarbonsäuren, Alkencarbonsäuren, Alkincarbonsäuren, Alkadiencarbonsäuren, Alkatriencarbonsäuren, Hydroxycarbonsäuren oder Ketocarbonsäuren aufgeführt. Geeignete Alkancarbonsäuren, Alkencarbonsäuren und Alkadiencarbonsäuren sind auch als Fettsäuren bekannt.

Ganz besonders bevorzugte Carboxylate sind die Anionen der C1 bis C10 Alkancarbonsäuren, insbesondere C1 bis C6 Alkancarbonsäuren, ganz besonders bevorzugt der Essigsäure (Acetat) und der Propionsäure (Propionat).

Bei der ionischen Flüssigkeit handelt es sich demnach besonders bevorzugt um Imidazoliumsalze der Formel I

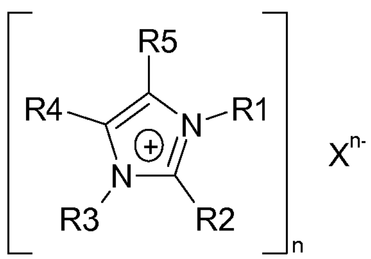

worin

R1 und R3 unabhängig voneinander für einen organischen Rest mit 1 bis 20 C-Atomen stehen

R2, R4, und R5 unabhängig voneinander für ein H-Atom oder für einen organischen Rest mit 1 bis 20 C-Atomen

stehen,

X für ein Carboxylat oder Phosphat steht, und

n für 1, 2 oder 3 steht.

R1 und R3 stehen vorzugsweise unabhängig voneinader für eine organische Gruppe, die 1 bis 10 C-Atome enthält. Besonders bevorzugt handelt es sich um eine Kohlenwasserstoffgruppe, welche keine weiteren Heteroatome aufweist, z.B. um eine gesättigte oder ungesättigte aliphatische Gruppe, eine aromatische Gruppe oder eine Kohlenwasserstoffgruppe, welche sowohl aromatische als auch aliphatische Bestandteile aufweist. Ganz besonders bevorzugt handelt es sich um eine C1 bis C10 Alkylgruppe, eine C1 bis C10 Alkenylgruppe, z.B. eine Allylgruppe, eine Phenylgruppe, eine Benzylgruppe. Insbesondere handelt es sich um eine C1 bis C4 Alkylgruppe, z.B. eine Methylgruppe, Ethylgruppe, Propylgruppe, i-Propylgruppe oder n-Butylgruppe.

R2, R4 und R5 stehen vorzugsweise unabhängig voneinander für ein H-Atom oder für eine organische Gruppe, die 1 bis 10 C-Atome enthält. Besonders bevorzugt handelt es sich bei R2, R4 und R5 um ein H-Atom oder um eine Kohlenwasserstoffgruppe, welche keine weiteren Heteroatome aufweist, z.B. um eine aliphatische Gruppe, eine aromatische Gruppe oder eine Kohlenwasserstoffgruppe, welche sowohl aromatische als auch aliphatische Bestandteile aufweist. Ganz besonders bevorzugt handelt es sich um ein H-Atom oder eine C1 bis C10 Alkylgruppe, eine Phenylgruppe oder eine Benzylgruppe. Insbesondere handelt es sich um ein h.Atom oder eine C1 bis C4 Alkylgruppe, z.B. eine Methylgruppe, Ethylgruppe, Propylgruppe, i-Propylgruppe oder n-Butylgruppe.

In einer besonderen Ausführungsform steht R 2 nicht für ein H-Atom sondern steht zwingend für den vorstehenden organischen Rest mit 1 bis 20 C- Atomen, insbesondere für eine C1 bis C4 Alkylgruppe.

n steht vorzugsweise für 1.

X steht vorzugsweise für ein Carboxylat, besonders bevorzugt für Acetat oder Propionat.

Als für das erfindungsgemäße Verfahren insbesondere geeignete ionische Flüssigkeiten seien solche mit 1,3-Dialkyl- und 1,2,3-Trialkylimidazolium-Kationen (mit Alkyl = C1 bis C8) und einem Acetat- oder Propionat-anion, vorzugsweise einem Acetat-anion genannt.

Ganz besonders bevorzugt sind die Propionate und insbesonderedie Acetate des 1-Methy-3-Ethylimidazoliums, 1,3-Diethylimidaoliums, 1,3-Dimethylimidazoliums, 1-Methyl-3-Butyl-Imidazoliums und 1-Ethyl-2,3-Dimethylimidazoliums.

Zu den Gemischen

Der Gehalt an ionischer Flüssigkeit im Gemisch beträgt mindestens 5 Gew.-%, bevorzugt mindestens 10 Gew.-%, besonders bevorzugt mindestens 20 Gew.-%, bezogen auf das gesamte Gemisch; das Verfahren eignet sich insbesondere auch für Gemische mit einem Gehalt von mindestens 30 bzw. 40 Gew.- % an ionischer Flüssigkeit.

Der Gehalt an ionischer Flüssigkeit ist im allgemeinen nicht größer als 95 Gew.-%, üblicherweise nicht größer als 90 Gew.-%, bzw. nicht größer als 80 Gew.-%.

Die ionischen Flüssigkeiten können ganz oder teilweise in dissoziierter Form oder in nicht dissoziierter Form (Kation/Anion-Paarbildung) vorliegen. Für die Durchführung des Verfahrens ist es nicht wesentlich, ob es in der flüssigen Phase zu einer Paarbildung von Anionen und Kationen der ionischen Flüssigkeit kommt oder ob die ionische Flüssigkeit z.B. bei Anwesenheit von Wasser oder sonstiger hydrophiler bzw. protischer Lösemittel ganz oder teilweise in dissoziierter Form vorliegt.

Für das erfindungsgemäße Verfahren eignen sich z.B. Gemische, die Verunreinigungen und Nebenprodukte durch den Herstellungsprozess oder die Verwendung der ionischen Flüssigkeit enthalten.

Die Gemische enthalten insbesondere Bestandteile mit einem Siedpunkt größer 200°C (1 bar) als Verunreinigungen, z.B. Salze, wie Alkali-acetate oder natürliche oder synthetische oligomere oder polymere Verbindungen, wie Lignin, Hemicellulose oder Oligosaccharide.

Gemische aus dem Herstellungsprozess

Für ionische Flüssigkeiten gibt es verschiedene Herstellungsverfahren. Bei diesen Verfahren werden üblicherweise Gemische erhalten, die neben der ionischen Flüssigkeit unerwünschte Nebenprodukte, Ausgangsprodukte und sonstige Verunreinigungen enthalten.

Für das erfindungsgemäße Verfahren geeignete Gemische sind z.B. solche die bei der Herstellung von Imidazoliumsalzen durch ein oder mehrstufige Umsetzung von Ausgangsverbindungen ausgewählt aus: $\alpha$-Dicarbonylverbindungen, Aminoverbindungen, Carbonylverbindungen, Ammoniak und Carbonatverbindungen erhalten werden.

Als Herstellverfahren ist z.B. die Carbonatmethode, welche in WO 2005/021484 und beschrieben ist.

Bei der Carbonatmethode werden Imidazoliumsalze durch Umsetzung einer $\alpha$-Dicarbonylverbindung , einer Carbonylverbindung (im allgemeinen Formaldehyd), einer Aminoverbindung und Ammoniak in einer ersten Stufe und einer anschließenden Umsetzung des Reaktionsprodukts in einer zweiten Stufe mit einem Carbonat (im allgemeinen Dimethylcarbonat) erhalten. Das nach der ersten Stufe erhaltene Gemisch enthält Nebenprodukte, die das Gemisch insgesamt dunkel bis schwarz erscheinen lassen.

Für das erfindungsgemäße Verfahren sind Gemische geeignet, die nach der ersten Stufe oder auch nach der zweiten Stufe des vorstehenden Herstellungsverfahrens erhalten werden.

Ein weiteres Herstellungsverfahren für Imidazoliumsalze wurde von Arduengo et al. (WO 91/14678, Arduengo-Verfahren) beschrieben. Bei diesem einstufigen Verfahren erfolgt die Herstellung durch Umsetzung einer $\alpha$-Dicarbonylverbindung, einer Carbonylverbindung (im allgemeinen Formaldehyd) und einer Aminoverbindung in Gegenwart einer Säure. Auch hier ist das erhaltene Gemisch aufgrund von Nebenprodukten dunkel bis schwarz gefärbt.

Für das erfindungsgemäße Verfahren sind Gemische geeignet, die nach diesem Herstellungsverfahren erhalten werden.

Gemische aus der Verwendung

Ebenso sind für das erfindungsgemäße Verfahren Gemische geeignet, die bei der Verwendung der ionischen Flüssigkeiten erhalten werden.

Ionische Flüssigkeiten werden bei ihrer Verwendung im allgemeinen nicht verbraucht sondern nur verunreinigt.

Bei der Verwendung der ionischen Flüssigkeiten werden daher Gemische erhalten, welche, die ionischen Flüssigkeiten und Verunreinigungen aus der jeweiligen Verwendung enthalten. Diese Gemische können mit dem erfindungsgemäßen verfahren wieder aufgearbeitet werden, so dass die ionische Flüssigkeit wieder verwendet werden kann.

Ionische Flüssigkeiten werden vielfältig als Lösemittel für Stoffe verwendet, die in anderen Lösemitteln nicht oder nur schwer löslich sind. Ionische Flüssigkeiten eignen sich z.B. als Lösemittel für Cellulose und Cellulose enthaltende Stoffe. Nach der jeweiligen Verwendung der Lösung, z.B. der Herstellung von Cellulose-Fasern aus der Lösung, werden Gemische erhalten, die ionische Flüssigkeiten und z.B. noch Cellulose, Lignin, Hemicellulosen enthalten. Das erfindungsgemäße Verfahren eignet sich daher insbesondere für Gemische, die nach Auflösen und Verarbeitung von Cellulose, bzw. Cellulose enthaltene Stoffe, erhalten werden.

Allgemeines zu Gemischen

Die bei dem erfindungsgemäßen Verfahren verwendeten Gemische enthalten leicht flüchtige Verbindungen nur in untergeordneten Mengen.

Unter leicht flüchtigen Verbindungen werden hier solche mit einem Siedpunkt kleiner 120°C, insbesondere kleiner 150°C bei Normaldruck (1 bar) verstanden.

Der Gehalt an leicht flüchtigen Verbindungen beträgt 0 bis 10 Gew.-%, insbesondere 0 bis 5 Gew.-% und besonders bevorzugt 0 bis 2 Gew.-%, bezogen auf das Gemisch.

Sofern leicht flüchtige Verbindungen zunächst im Gemisch vorhanden sind, werden sie vor Durchführung des erfindungsgemäßen Verfahrens weitgehend entfernt, so dass ihr Gehalt im Gemisch maximal 10 Gew.-%, insbesondere maximal 5 Gew.-%, insbesondere maximal 2 Gew.-% beträgt (siehe oben), besonders bevorzugt werden sie vollständig entfernt.

Der Zusatz einer starken Base, wie er z.B. in DE 103 33 239 zur Herstellung von gereinigten Imidazoliumsalzen beschrieben ist, ist im Rahmen dieser Erfindung nicht notwendig. Dem Gemische wird daher entsprechend vorzugsweise keine derartige starke Base ($pK_B$ kleiner 0 bei 1 bar, 21°C, gemessen in Wasser) oder überhaupt keine Base zugesetzt.

Zur Destillation

Wesentliches Merkmal des erfindungsgemäßen Destillationsverfahrens ist, dass bei der Destillation der Abstand der Fläche, über welche die Destillationswärme zugeführt wird (Verdampferoberfläche) zu der Fläche, an der die Kondensation erfolgt (Kondensatoroberfläche), an mindestens einem Punkt geringer als 50 cm ist.
Die Verdampfer- und Kondensatoroberfläche selbst haben dabei mindestens eine Längenabmessung größer 50 cm, d.h. diese Oberflächen sind groß im Vergleich zu dem Abstand der Flächen.

Destillationsverfahren mit einem geringen Abstand zwischen Verdampfer- und Kondensatoroberfläche sind als Molekulardestillation bekannt. Bei der Molekulardestillation ist der Abstand zwischen Verdampfer- und Kondensatoroberfläche im allgemeinen kleiner als die mittlere freie Weglänge der zu destillierenden Verbindungen. Dazu werden die apparative Geometrie und die Verfahrensparameter (Druck und Temperatur) entsprechend gewählt.

Die apparative Anordnung der Kondensatoroberfläche zur Verdampferoberfläche kann in beliebiger geometrischer Form ausgestaltet sein. Wesentlich ist, dass sie direkt gegenüberliegen, so dass die Moleküle von der Verdampferoberfläche ungehindert zur Kondensatoroberfläche gelangen.
In Betracht kommt z.B. eine planparallele Anordnung der beiden Flächen oder auch eine zylinderförmige Anordnung, wobei zwei Zylinder ineinander gesetzt sind und die direkt gegenüberliegenden Oberflächen der beiden Zylinder die Verdampfer- bzw. Kondensatoroberfläche bilden.

Die Verdampferoberfläche wird in geeigneter Weise beheizt, im allgemeinen durch Vorrichtungen auf der Rückseite, entsprechend wird die Kondensatoroberfläche im allgemeinen entsprechend gekühlt, ebenfalls durch Vorrichtungen auf der Rückseite.

Der Abstand der Verdampferoberfläche zu der Kondensatoroberfläche ist an mindestens einem Punkt geringer als 50 cm, insbesondere geringer als 40 cm, besonders bevorzugt geringer als 30 cm.

Der Abstand der Verdampferoberfläche zu der Kondensatoroberfläche ist insbesondere geringer als die mittlere freie Weglänge der ionischen Flüssigkeit in der Gasphase bei der gewählten Temperatur und dem gewählten Druck. Die mittlere freie Weglänge ($\lambda_M$) lässt sich nach bekannten Verfahren bestimmen und ergibt sich der Gleichung:

$$\lambda_M = const \ x \ T/ (p \, _{\sigma}{}^2)$$

worin die Symbole folgende Bedeutung haben:

T: Temperatur
P: Druck
$\sigma$: Stoßquerschnitt des Ionenpaares (ionische Flüsssigkeit), entspricht Querschnittsfläche des Ionenpaares

Bevorzugt geeignete Vorrichtungen sind so ausgestaltet, dass mindestens 10 Flächenprozent, besonders bevorzugt mindestens 20 Flächenprozent, ganz besonders bevorzugt mindestens 30 oder gar mindestens 50 Flächenprozent der Verdampferoberfläche zu der Kondensatoroberfläche den vorstehenden Mindestabstand aufweisen.

Die Verdampferoberfläche und die Kondensatoroberfläche können z.B. bei Apparaturen im technischen Maßstab jeweils größer als 0,5 $m^2$ sein.

Bei dem erfindungsgemäßen Verfahren wird die ionische Flüssigkeit aus dem Gemisch abgetrennt und als Destillat von der Kondensatoroberfläche abgezogen. Der Rückstand verbleibt auf der Verdampferoberfläche entfernt. Geeignete Vorrichtungen sind z.B. derart ausgestaltet, dass der Rückstand von der Verdampferoberfläche abläuft und aufgefangen wird, entsprechend läuft die ionische Flüssigkeit von der Kondensatoroberfläche ab und wird als Destillat erhalten.

Die Oberflächentemperatur des Verdampfers und der Druck werden vorzugsweise so gewählt, dass der Abstand zwischen Verdampfer- und Kondensatoroberfläche kleiner ist als die freie Weglänge der abzutrennenden ionischen Flüssigkeit in der Gasphase.

Die Oberflächentemperatur beträgt vorzugsweise 110 bis 300°C, besonders bevorzugt 130 bis 280°C und ganz besonders bevorzugt 140°C bis 260°C.

Der Druck in dem Bereich zwischen Verdampferoberfläche und Kondensatoroberfläche beträgt vorzugsweise 0,0001 bis 10 mbar, bevorzugt 0,001 bis 5 mbar, besonders bevorzugt 0,05 bis 5 mbar.

Das erhaltene Destillat kann z.B. zu mehr als 95 Gew.-%, besonders bevorzugt zu mehr als 97 Gew.-%" ganz besonders bevorzugt zu mehr als 99 Gew.-% aus der ionischen Flüssigkeit bestehen. Insbesondere sind mit dem Verfahren auch Destillate erhältlich, welche zu mehr als 99,5 bzw. zu mehr als 99,8 Gew.-% aus der ionischen Flüssigkeit bestehen.

Mit dem erfindungsgemäßen Verfahren können daher ionische Flüssigkeiten aus beliebigen Gemischen in hoher Reinheit gewonnen werden.

Das Verfahren kann kontinuierlich oder diskontinuierlich durchgeführt werden.

Beispiele:

Verwendete ionische Flüssigkeiten (IL)

| Abkürzungen | chemische Bezeichnung |
|---|---|
| BMIM OAc | 1-Butyl-3-Methyl-Imidazolium Acetat |
| EMIM OAc | 1-Ethyl-3-Methyl-Imidazolium Acetat |
| EEIM OAc | 1,3-Diethyl-Imidazolium Acetat |
| BMIM OProp | 1-Butyl-3-Methylimidazolium Propionat |

(fortgesetzt)

| | |
|---|---|
| BMIM Cl | 1-butyl-3-Methyl-Imidazolium Chlorid |
| EMIM DEP | 1-Ethyl-3-Methyl-Imidazolium Diethylphosphat |

Vor allen Destillationsversuchen wird die IL durch Rühren bei 120°C und 0,1 mbar für 16 h von Leichtsiedern befreit, um ein Schäumen und Verspritzen während der Molekulardestillation zu vermeiden.

Der Versuchsaufbau ist in der beigefügten Skizze beschrieben. Das Destillat läuft an der Kondensatoroberfläche ab und wird unten gesammelt, entsprechend läuft der Rückstand an der Verdampferoberfläche ab und wird unten gesammelt.

Beispiel 1:

Bestimmung der Destillationstemperaturfür EMIM OAc

Ca. 100 ml EMIM Oac werden in das Zulaufgefäß der Molekulardestillation eingefüllt, die Apparatur mit Stickstoff inertisiert und ein Vakuum von 0,05 mbar angelegt. Die Verdampferfläche wird auf die angegebene Temperatur erwärmt und der Zulauf gestartet mit einer Geschwindigkeit von ca. 50 ml/h.

Nachdem alles EMIM OAc zugelaufen ist, wird abgekühlt, mit Stickstoff belüftet und das Gewichtsverhältnis von Destillat und Rückstand, sowie die Reinheit beider Teile durch H-NMR und die Masse der Zersetzungsprodukte in der Kühlfalle bestimmt.

Die Bedingungen werden dann als geeignet angesehen, wenn die Massenanteile von Destillat zu Rückstand mindestens 8:2 betragen und weniger als 10% des Ausgangsmaterials zersetzt sind. Prinzipiell lässt sich der Destillationsgrad bei gegebenem Druck durch Temperaturerhöhung (Obergrenze: zu starke thermische Zersetzung) oder bei gegebener Temperatur durch Druckabsenkung (technische Grenze durch erreichbaren Mindestdruck) steigern.

Für EMIM OAc wurden folgende Resultate erhalten:

| Temp. Verdampfer | Destillat | | Rückstand | |
|---|---|---|---|---|
| °C | Masse g | Reinheit (H_NMR) | Masse g | Reinheit (HNMR) |
| 100 | < 1 | >95% | 115 | >95% |
| 130 | 45 | >95% | 55 | >95% |
| 150 | 96 | >95% | 20 | >95% |
| 170 | 108 | >95% | 10 | >95% |

Bei keinem der Experimente wurde in der Kühlfalle zur Vakuumpumpe Destillat gefunden.

Beispiel 2:

Destillation von EEIM OAc

Rohes EEIM OAc wird durch Reaktion von Formaldehyd, Glyoxal und Ethylamin in Wasser mit Essigsäure hergestellt und durch Einengen bei zunächst 10 mbar / 80°C, danach wie oben angegeben bei 0,1 mbar/120°C von Leichtsiedern befreit. Das Rohprodukt ist tief dunkelbraun.

200 g dieses Rohproduktes werden innerhalb von 2 Stunden bei 0,05 mbar und 170°C Wandtemperatur (Verdampferoberfläche) in die Molekulardestillation gefahren.

Es werden 190 g farbloses klares Destillat erhalten, bei dem es sich nach H-NMR um reines (>95%) EEIM OAc handelt, und 10 g eines schwarzen, teerartigen Rückstandes. In der Kühlfalle werden keine flüchtigen Bestandteile aufgefangen.

Beispiel 3:

Destillation von EMIM OAc

Als Edukt werden 200 g EMIM OAc verwendet, das zuvor fünfmal zur Auflösung von Cellulose (Pulp der Fa.

Tembec Inc. Type 10A) und Rückgewinnung der Cellulose durch Verdünnen mit der 10-fachen Menge Wasser verwendet wurde (siehe WO 03/029329). Das EMIM OAc wurde nach jeder Ausfällung aus derwässrigen überstehenden Lösung durch Abdestillieren des Wassers bei 120°C / 1 mbar wiedergewonnen und ohne weitere Reinigung wieder eingesetzt. Vor der Molekulardestillation wurde es wie oben beschrieben von Leichtsiedern befreit.

Das zur Destillation gelangende EMIM OAc enthält ca. 6 Gew-% Nebenkomponenten (z.B. Lignin) aus der Cellulose und ist gelbbraun verfärbt (Farbzahl 16 nach Gradener).

200 g dieses EMIM OAc werden innerhalb von 4 Stunden bei 0,05 mbar und 170°C Wandtemperatur der Molekulardestillation zugefahren.

Als Destillat werden 170 g farbloses klares EMIM OAc erhalten, das nach H-NMR eine Reinheit >95% aufweist. Der Rückstand sind 30 g eines schwarzen, teerartigen, nicht weiter charakterisierten Materials.

Beispiel 4:

Destillation verschiedener IL's

Jeweils ca. 100 g der in der Tabelle angegebenen IL werden innerhalb von 2 Stunden der Molekulardestillation zugefahren bei 0,05 mbar und der angegebenen Wandtemperatur. Das Massenverhältnis von Destillat und Rückstand wurde ermittelt.

| IL | Wandtemp. | Destillat | Rückstand | |
|---|---|---|---|---|
| | °C | Gew-% | Gew-% | |
| BMIM OAc | 170 | 90 | 10 | Beide >95% BMIM OAc (H-NMR) |
| EEIM OAc | 170 | 90 | 10 | Beide >95% EEIM OAc (H-NMR) |
| BMIM OProp | 170 | 90 | 10 | Beide >95% BMIM OProp (H-NMR) |
| EMIM DEP | 170 | 0 | 100 | Beide >95% EMIM DEP (H-NMR) |
| EMIM DEP | 240 | 12 | 88 | Beide >95% EMIM DEP (H-NMR) |
| EMIM DEP | 250 | 54 | 46 | Beide >95% EMIM DEP (H-NMR) |
| BMIM Cl * | 170 | 0 | 100 | >95% BMIM Cl (H-NMR) |
| BMIM Cl * | 250 | 13 | 85 | Dest. > 50% Butyl- und Ethylimidazol |

**Patentansprüche**

1. Verfahren zur Destillation von Gemischen, welche Salze mit einem Schmelzpunkt kleiner 200°C bei 1 bar (ionische Flüssigkeiten) enthalten, **dadurch gekennzeichnet, dass**

   - das Kation der ionischen Flüssigkeit ein heterocyclisches Ringsystem mit mindestens einem Stickstoffatom enthält und alle Stickstoffatome des heterocyclischen Ringsystems eine organische Gruppe als Substituenten haben
   - es sich bei dem Anion der ionischen Flüssigkeit um eine Verbindung mit mindestens einer Carboxylatgruppe (kurz Carboxylat) oder mindestens einer Phosphatgruppe (kurz Phosphat) handelt,
   - der Gehalt an ionischer Flüssigkeit im Gemisch mindestens 5 Gew. % beträgt,
   - der Gehalt an leicht flüchtige Verbindungen (Siedepunkt kleiner 120°C, 1 bar) 0 bis 10 Gew.%, bezogen auf das Gemisch, beträgt
   - bei der Destillation der Abstand der Fläche, über welche die Destillationswärme zugeführt wird (Verdampferoberfläche) zu der Fläche, an der die Kondensation erfolgt (Kondensatoroberfläche), an mindestens einem Punkt geringer als 50 cm ist, wobei die Verdampfer- und Kondensatoroberfläche selbst mindestens eine Längenabmessung größer 50 cm haben,
   - die ionische Flüssigkeit als Destillat von der Kondensatoroberfläche abgezogen wird und
   - es sich um eine Molekulardestillation handelt.

2. Verfahren gemäß Anspruch 1, **dadurch gekennzeichnet, dass** es sich bei dem Kation um ein Imidazolium-kation handelt.

**3.** Verfahren gemäß Anspruch 1 oder 2, **dadurch gekennzeichnet, dass** es sich bei den Substituenten der Stickstoffatome in dem heterocyclischen Ringsystem um C1 bis C10 Alkylgruppen handelt.

**4.** Verfahren gemäß einem der Ansprüche 1 bis 3, **dadurch gekennzeichnet, dass** es sich bei der ionischen Flüssigkeit um Imidazoliumsalze der Formel I

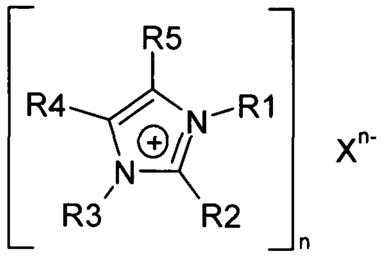

handelt, worin

R1 und R3 unabhängig voneinander für einen organischen Rest mit 1 bis 20 C-Atomen stehen
R2, R4, und R5 unabhängig voneinander für ein H-Atom oder für einen organischen Rest mit 1 bis 20 C-Atomen stehen,
X für ein Carboxylat steht, und
n für 1, 2 oder 3 steht,

**5.** Verfahren gemäß Anspruch 4, **dadurch gekennzeichnet, dass** R2 für einen organischen Rest mit 1 bis 20 C-Atomen steht.

**6.** Verfahren gemäß einem der Ansprüche 1 bis 5, **dadurch gekennzeichnet, dass** die Gemische zu 10 bis 95 Gew. % aus der ionischen Flüssigkeit bestehen.

**7.** Verfahren gemäß einem der Ansprüche 1 bis 6, **dadurch gekennzeichnet, dass** es sich um Gemische handelt, die bei der Herstellung von ionischen Flüssigkeiten durch ein oder mehrstufige Umsetzung von Ausgangsverbindungen ausgewählt aus: $\alpha$-Dicarbonylverbindungen, Aminoverbindungen, Carbonylverbindungen, Ammoniak und Carbonatverbindungen erhalten werden.

**8.** Verfahren gemäß einem der Ansprüche 1 bis 6, **dadurch gekennzeichnet, dass** es sich um Gemische handelt, die bei der Verwendung der ionischen Flüssigkeiten erhalten werden.

**9.** Verfahren gemäß einem der Ansprüche 1 bis 8, **dadurch gekennzeichnet, dass** die Gemische weitere Bestandteile mit einem Siedpunkt größer 200°C (1 bar) als Verunreinigungen enthalten, z. B. Salze, wie Alkali-acetate oder natürliche oder synthetische oligomere oder polymere Verbindungen, wie Lignin, Hemicellulose oder Oligosaccharide.

**10.** Verfahren gemäß einem der Ansprüche 1 bis 9, **dadurch gekennzeichnet, dass** mindestens 10 Flächenprozent der Verdampferoberfläche zu der Kondensatoroberfläche einen Abstand kleiner 50 cm haben.

**11.** Verfahren gemäß einem der Ansprüche 1 bis 10, **dadurch gekennzeichnet, dass** die Verdampferoberfläche und die Kondensatoroberfläche jeweils größer als 0,5 $m^2$ sind.

**12.** Verfahren gemäß einem der Ansprüche 1 bis 11, **dadurch gekennzeichnet, dass** die Destillation bei einer Oberflächentemperatur des Verdampfers von 110° C bis 300°C und einem Druck von 0,0001 bis 10 mbar durchgeführt wird.

**13.** Verfahren gemäß einem der Ansprüche 1 bis 12, **dadurch gekennzeichnet, dass** das erhaltene Destillat zu mehr als 97 Gew. % aus der ionischen Flüssigkeit besteht.

## Claims

1. A method of distilling mixtures comprising salts having a melting point of less than 200°C at 1 bar (ionic liquids), wherein

   - the cation of the ionic liquid comprises a heterocyclic ring system having at least one nitrogen atom and all nitrogen atoms of the heterocyclic ring system have an organic group as substituent,
   - the anion of the ionic liquid is a compound having at least one carboxylate group (carboxylate for short) or at least one phosphate group (phosphate for short),
   - the content of ionic liquid in the mixture is at least 5% by weight,
   - the content of volatile compounds (boiling point less than 120°C, 1 bar) is from 0 to 10% by weight, based on the mixture,
   - the distance from the surface via which the heat of distillation is introduced in the distillation (vaporizer surface) to the surface at which condensation takes place (condenser surface) is less than 50 cm at at least one point, with the vaporizer surface and condenser surface themselves having at least one length dimension of greater than 50 cm,
   - the ionic liquid is removed as a distillate from the condenser surface and
   - it is a molecular distillation.

2. The method according to claim 1, wherein the cation is an imidazolium cation.

3. The method according to claim 1 or 2, wherein the substituents on the nitrogen atoms in the heterocyclic ring system are C1-C10-alkyl groups.

4. The method according to any of claims 1 to 3, wherein the ionic liquid is an imidazolium salt of the formula I

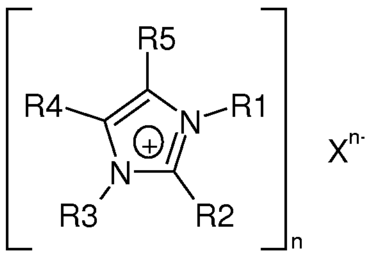

   where

   R1 and R3 are each, independently of one another, an organic radical having from 1 to 20 carbon atoms,
   R2, R4, and R5 are each, independently of one another, an H atom or an organic radical having from 1 to 20 carbon atoms,
   X is a carboxylate and
   n is 1, 2 or 3.

5. The method according to claim 4, wherein R2 is an organic radical having from 1 to 20 carbon atoms.

6. The method according to any of claims 1 to 5, wherein the mixtures comprise from 10 to 95% by weight of the ionic liquid.

7. The method according to any of claims 1 to 6, wherein the mixtures are mixtures which are obtained in the preparation of ionic liquids by single-stage or multistage reaction of starting compounds selected from among: $\alpha$-dicarbonyl compounds, amino compounds, carbonyl compounds, ammonia and carbonate compounds.

8. The method according to any of claims 1 to 6, wherein the mixtures are mixtures which are obtained in the use of ionic liquids.

9. The method according to any of claims 1 to 8, wherein the mixtures comprise further constituents having a boiling point above 200°C (1 bar) as impurities, e.g. salts such as alkali metal acetates or natural or synthetic oligomeric

or polymeric compounds such as lignin, hemicellulose or oligosaccharides.

**10.** The method according to any of claims 1 to 9, wherein at least 10 percent by area of the vaporizer surface is at a distance of less than 50 cm from the condenser surface.

**11.** The method according to any of claims 1 to 10, wherein the vaporizer surface and the condenser surface are each larger than 0.5 $m^2$.

**12.** The method according to any of claims 1 to 11, wherein the distillation is carried out at a surface temperature of the vaporizer of from 110°C to 300°C and a pressure of from 0.0001 to 10 mbar.

**13.** The method according to any of claims 1 to 12, wherein the distillate obtained comprises more than 97% by weight of the ionic liquid.

## Revendications

**1.** Procédé de distillation de mélanges qui contiennent des sels ayant un point de fusion inférieur à 200 °C à 1 bar (liquides ioniques), **caractérisé en ce que**

- le cation du liquide ionique contient un système cyclique hétérocyclique contenant au moins un atome d'azote et tous les atomes d'azote du système cyclique hétérocyclique comprennent un groupe organique en tant que substituant,
- l'anion du liquide ionique est un composé contenant au moins un groupe carboxylate (en abrégé carboxylate) ou au moins un groupe phosphate (en abrégé phosphate),
- la teneur en liquide ionique dans le mélange est d'au moins 5 % en poids,
- la teneur en composés volatils (point d'ébullition inférieur à 120 °C, 1 bar) est de 0 à 10 % en poids, par rapport au mélange,
- lors de la distillation, la distance entre la surface par le biais de laquelle la chaleur de distillation est introduite (surface de l'évaporateur) et la surface sur laquelle la condensation a lieu (surface du condensateur) est inférieure à 50 cm à au moins un point, la surface de l'évaporateur et la surface du condensateur ayant elles-mêmes au moins une dimension longitudinale supérieure à 50 cm,
- le liquide ionique est soutiré en tant que distillat de la surface du condensateur, et
- il s'agit d'une distillation moléculaire.

**2.** Procédé selon la revendication 1, **caractérisé en ce que** le cation est un cation imidazolium.

**3.** Procédé selon la revendication 1 ou 2, **caractérisé en ce que** les substituants des atomes d'azote dans le système cyclique hétérocyclique sont des groupes alkyle en C1 à C10.

**4.** Procédé selon l'une quelconque des revendications 1 à 3, **caractérisé en ce que** le liquide ionique correspond à des sels d'imidazolium de formule I

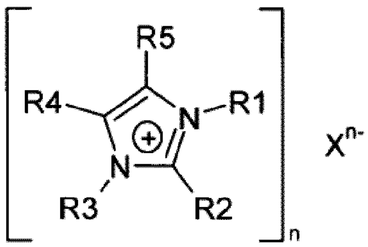

dans laquelle

R1 et R3 représentent indépendamment l'un de l'autre un radical organique de 1 à 20 atomes C,
R2, R4 et R5 représentent indépendamment les uns des autres un atome H ou un radical organique de 1 à 20 atomes C,
X représente un carboxylate, et

n représente 1, 2 ou 3.

5. Procédé selon la revendication 4, **caractérisé en ce que** R2 représente un radical organique de 1 à 20 atomes C.

6. Procédé selon l'une quelconque des revendications 1 à 5, **caractérisé en ce que** les mélanges sont constitués de 10 à 95 % en poids du liquide ionique.

7. Procédé selon l'une quelconque des revendications 1 à 6, **caractérisé en ce qu'**il s'agit de mélanges obtenus lors de la fabrication de liquides ioniques par mise en réaction à une ou plusieurs étapes de composés de départ choisis parmi : les composés d'$\alpha$-dicarbonyle, les composés amino, les composés de carbonyle, l'ammoniac et les composés de carbonate.

8. Procédé selon l'une quelconque des revendications 1 à 6, **caractérisé en ce qu'**il s'agit de mélanges obtenus lors de l'utilisation des liquides ioniques.

9. Procédé selon l'une quelconque des revendications 1 à 8, **caractérisé en ce que** les mélanges contiennent d'autres constituants ayant un point d'ébullition supérieur à 200 °C (1 bar) en tant qu'impuretés, p. ex. des sels, tels que des acétates alcalins, ou des composés oligomères ou polymères naturels ou synthétiques, tels que la lignine, l'hémicellulose ou des oligosaccharides.

10. Procédé selon l'une quelconque des revendications 1 à 9, **caractérisé en ce qu'**au moins 10 pourcent de surface de la surface de l'évaporateur est à une distance inférieure à 50 cm de la surface du condensateur.

11. Procédé selon l'une quelconque des revendications 1 à 10, **caractérisé en ce que** la surface de l'évaporateur et la surface du condensateur sont chacune d'une taille supérieure à 0,5 $m^2$.

12. Procédé selon l'une quelconque des revendications 1 à 11, **caractérisé en ce que** la distillation est réalisée à une température de surface de l'évaporateur de 110 °C à 300 °C et à une pression de 0,0001 à 10 mbar.

13. Procédé selon l'une quelconque des revendications 1 à 12, **caractérisé en ce que** le distillat obtenu est constitué de plus de 97 % en poids du liquide ionique.

Figur 1

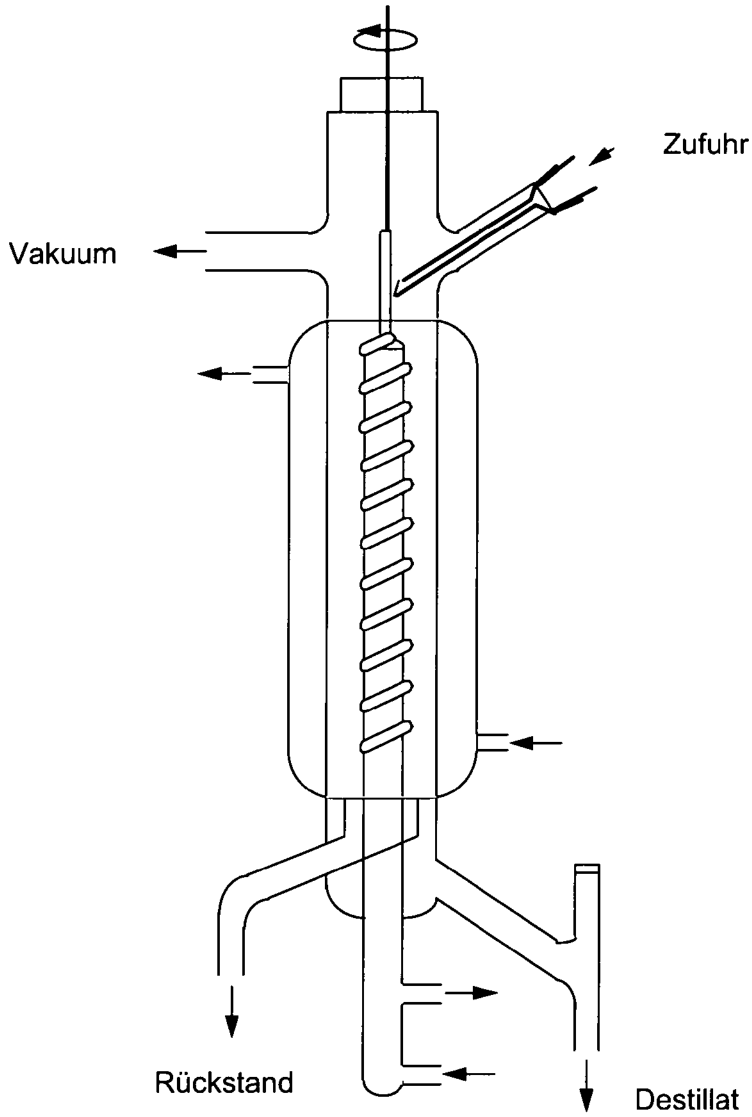

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2005068404 A
- WO 2005021484 A
- WO 9114678 A
- DE 10333239
- WO 03029329 A


### In der Beschreibung aufgeführte Nicht-Patentliteratur


- **MARTYN J. EARLE ; JOSE M.S.S. ESPERANCA et al.** *Nature,* 2006, vol. 439, 831-834
- **DOUGLAS R. MACFARLANE ; JENNIFER M. PRINGLE et al.** *Chem.Commun.,* 2006, 1905-1917